(19) Europäisches Patentamt
European Patent Office
Office européen des brevets

(11) **EP 2 813 174 A1**

(12) **EUROPÄISCHE PATENTANMELDUNG**

(43) Veröffentlichungstag:
**17.12.2014   Patentblatt 2014/51**

(51) Int Cl.:
*A61B 5/00* (2006.01)     *A61B 5/0205* (2006.01)
*A61B 5/1455* (2006.01)   *A61B 5/024* (2006.01)

(21) Anmeldenummer: **13171362.0**

(22) Anmeldetag: **11.06.2013**

(84) Benannte Vertragsstaaten:
**AL AT BE BG CH CY CZ DE DK EE ES FI FR GB
GR HR HU IE IS IT LI LT LU LV MC MK MT NL NO
PL PT RO RS SE SI SK SM TR**
Benannte Erstreckungsstaaten:
**BA ME**

(71) Anmelder: **Spiess Media Systems Asc, Corp.
22589 Hamburg (DE)**

(72) Erfinder:
• **Frönicke, Max Carl August
40477 Düsseldorf (DE)**

• **Zembold, Nina
51381 Leverkusen (DE)**
• **Zembold, Otmar
22589 Hamburg (DE)**

Bemerkungen:
Die Patentansprüche 20 und 21 gelten als fallen gelassen, da die entsprechenden Anspruchsgebühren nicht entrichtet wurden (R. 45 (3) EPÜ).

(54) **Tragbares modulares Sensorsystem**

(57)   Ein mobiles modulares System zur nicht-invasiven, simultanen und kontinuierlichen Messung und Analyse von Vitaldaten zur Bestimmung des physiologischen Gesundheitszustandes einer Person beinhaltet ein tragbares Bandsystem mit über ein Bussystem verbundenen, modularen Sensoreinheiten. Dabei kommen schwerpunktmäßig spektroskopische Verfahren zum Einsatz, aber auch weitere physiologische Sensoren sowie Sensoren zur Erfassung von Umweltbedingungen können Verwendung finden.

EP 2 813 174 A1

**Beschreibung**

Bei gesundheitlichen Schwachstellen und Gesundheitsproblemen eines Menschen wird überwiegend mit invasiv-labormedizinischen und/oder empirisch-medizinischen Diagnose-Methoden versucht die Ursachen zu erkennen und darauf zu reagieren. Allerdings ist auf diese Weise ein vorsorgliches und zur Früherkennung von nicht manifestierten körperlichen Schwachstellen geeignetes automatisches Monitoring gesundheitlich gefährdeter Menschen bzw. Personen kaum und wenn nur stationär und/oder mit großem gerätetechnischen Aufwand möglich. Der Erfindung liegt daher die Aufgabe zugrunde, ein System/Verfahren, ein Multisensor-Bus-System und eine Infrastruktur zu schaffen und/oder zu nutzen, um ein variables, fallweise anwendbares Monitoring zu ermöglichen. Das ist heute mit der Höchstintegration geeigneter Mikro- und Nano-Sensoren erfindungsgemäß dadurch zu realisieren, dass vielfältige Messung mithilfe eines kleinen, mobilen, tragbaren, modular aufgebauten Multisensor-Bus-System in Form eines flexiblen und/oder halbstarren Bus-Bandes, die simultane Erfassung geeigneter Vitalparameter an einer bestimmten Stelle des menschlichen Körpers - je nach gewünschtem Messdatenspektrums - durchgeführt wird. Mithilfe von optischen, akustischen, elektromagnetischen, druck-, temperatur- und bewegungssensitiv ermittelten Werten und deren Weiterverarbeitung und Auswertung über aktive elektronischen Filter wird erfindungsgemäß eine verbesserte neutrale Analyse und objektivierte Diagnose von gesundheitlichen Schwachstellen und die Früherkennung von gesundheitlichen Fehlentwicklungen im Organismus eines Menschen ermöglicht.

Die Erfindung betrifft somit ein mobiles, mit verschiedenen Komponenten modular über eine Bus-Struktur aufgebautes tragbares Multisensor-Bus-system kombiniert mit einem Verfahren zur nichtinvasiven Überwachung von funktionellen und/oder pathologischen Veränderungen im psychischen Verhalten und/oder im physischen Organismus eines Menschen in Kombination mit der Übertragung standardisierter Werte an - und/oder die Verarbeitung der erfassten Vitalsignale in - BigData-basierten Datenbanksysteme sowie deren KI-Auswertung zur verbesserten Vital-Analyse und für eine objektivierte Diagnose.

Situation allgemein

Ein zunehmender Teil des aktuellen Gesundheits- und Medizinbetriebes beschäftigt sich mit Vorsorgeuntersuchungen und Früherkennung. Auch die Bundesregierung möchte die Gesundheitsvorsorge mit einem sogenannten Präventionsgesetz fördern, wie ein vom Bundeskabinett im März 2013 verabschiedeter Gesetzentwurf belegt.

Die empfohlenen Maßnahmen fangen unmittelbar nach der Geburt an und erstrecken sich bis ins hohe Alter und schließen auch das betriebliche Vorsorge-Gesundheitswesen ein.

Ziel ist die frühzeitige Erkennung von gesundheitlichen Schwachstellen eventuell verbunden mit der Erkenntnis über eine negative Entwicklung des Gesundheitszustandes, um geeignete Vorsorgemaßnahmen ergreifen zu können.

Dabei werden die derzeit praktizierten Früherkennungsmaßnahmen punktuell auch zeitperiodisch (z.B. jährlich) durchgeführt und nur bestimmte standardisierte körperliche Vitalparameter eines Menschen erfasst, die aber über seine individuelle Gesamtverfassung nur eben zum gegebenen Zeitpunkt etwas aussagen. Nicht selten werden durch die Untersuchungen (Mammographie, Abstriche, Spiegelungen, Sonographien) aber auch Ängste geschürt, wenn sie unklare oder grenzwertige Befunde ergeben, die kurzfristige aber immer nur punktuell wiederholte Kontrolluntersuchungen erfordern. Außerdem gibt es Menschen, die regelmäßig zur "Vorsorge" gehen, sich untersuchen und Blut abnehmen lassen und die sich in Sicherheit wähnen, wenn nichts Krankhaftes festgestellt wird. Auf der anderen Seite bestehen nicht selten nur zeitweise auftretende Beschwerden, ohne dass sich mit Labor, Röntgen, CT oder anderen diagnostischen Mess- und Analyse-Methoden eine Ursache erkennen lässt.

Es gibt also zweifelsfrei eine Grauzone zwischen Gesundheit und Krankheit, die sich trotz aller gegenwärtigen aber immer nur punktuellen Diagnostik schlecht erfassen lässt.

Darüber hinaus bringen die vorhandenen vielfältigen und komplexen Umwelt- und Arbeitsweltbelastungen zunehmende Überforderung des menschlichen Organismus mit der Konsequenz einer ständig steigenden Anzahl von psychophysiologischen Burnouts.

Zudem sind das Bewusstsein und die Sensitivität des Menschen mit Bezug auf körperliche Signale sehr unterschiedlich ausgeprägt. Das soll erfindungsgemäß verbessert werden.

Status quo

Mit der Änderung der Lebensgewohnheiten, den demographischen Veränderungen aber auch der rapiden Zunahme der Übergewichtigen, der Diabetiker und Gestressten ist eine enorme Steigerung beispielsweise allein nur der kardialen Notfälle vorprogrammiert. Es besteht deshalb ein ständig zunehmender Handlungsbedarf an effektiven und leistungsfähigen Vorsorge- und Notfallmaßnahmen. So ist beispielsweise die Situation "Herzinfarkt" ein meist plötzliches, unvorhersehbares, schicksalhaftes Ereignis. Das Versagen unseres wichtigsten Motors Herz kann jeden Menschen in jedem Alter treffen. Urplötzlich und überall, und oftmals tödlich. Prominente wie der Regisseur Bernd Eichinger, der Schauspieler Dirk Bach und Millionen andere sind überzeugende Beispiele. Der Herzinfarkt ist die häufigste Todesursache weltweit.

In China stirbt alle 10 Sekunden ein Mensch an Herzversagen, in den USA alle 45 Sekunden in Deutschland alle 120 Sekunden.

Quelle: WHO-Report 29. Sept. 2012, Global atlas ISBN 9789241564373, Cardiovascular diseases

Allein in Deutschland gibt es jedes Jahr über 300 000 Herzinfarkte. Davon kommen über die Hälfte - 150 000 - bereits tot in der Klinik an. Weitere ca. 20 Prozent versterben im Laufe des Jahres. Quellen: Kongressbericht 14.10.2012, Deutsche Gesellschaft für Kardiologie, Todesursachenstatistik, Statistisches Bundesamt 2011, GEDA Faktenblätter, Kap.6.11 Robert Koch Institut 2012

Alle neueren phantastischen medizinisch-technischen und nano-biologischen Errungenschaften ein Herz zu reparieren sind aber wertlos, wenn der Motor "Herz" bereits stillsteht.

Die Erfindung bezieht sich nicht nur in diesem konkreten, speziell kardiovaskulären Fall auf eine System und Verfahren mit spezifischer Datenerfassungs- und besonders gesicherter Kommunikationsinfrastruktur zur Analyse und Diagnose des individuellen Herz-Gesundheitszustandes eines Menschen. Hierzu gehört vor allem der Vergleich der erfassten Herz-Vitalparameter in Form aufbereitete Messdaten als eine Ereignis-Erkennung mit einem vorhandenen Datensatz auf einem zentralen Datenbank-Server, einschließlich der automatischen Erzeugung eines Notsignals auf der Grundlage eines Notfalls. Insbesondere bezieht sich die Erfindung auf ein System und Verfahren mit geeigneter Datenaufbereitung zur Erleichterung der Kommunikation zwischen der systemischsimultanen Bus-Sensor-Erfassungseinrichtung und einem Notfall-Dienstleister sowie verschiedene weitere Funktionen, wie beispielsweise die Ortung des Nutzers bzw. Anwenders oder eine aufbereitete Messdaten-Aggregation, wie sie in den weiter unten spezifizierten Ansprüchen abgeführt worden sind.

Bei den durchschnittlich 300.000 Herzinfarkte pro Jahr in Deutschland wird angenommen, man hätte die meisten der daraus resultierenden 150.000 Todesfälle verhindern können, wenn rechtzeitig entsprechende Hilfe in einer fristgerechten Weise möglich gewesen wäre.

Der Erfindung liegt nicht nur in diesem hervorgehobenen Fall eines Herzinfarktes die Aufgabe zugrunde, eine Vorrichtung zur nichtinvasiven Detektion pathologischer Veränderungen im Herz-Kreislauf-System sowie von Durchblutungsstörungen zur Verfügung zu haben, die im Vorfeld ein systematisches und simultanes Monitoring geeigneter Vitalparameter erlaubt ohne dass die Person explizit und aktiv erforderliche Maßnahmen einleiten und/oder ergreifen muss.

Erfindungsgemäß kann und soll deshalb das Bus-Sensor-System auch zur Detektion pathologischer Veränderungen im Herz-Kreislauf-System allgemein Verwendung finden aber unter anderem mit einem kardiovaskulären Schwerpunkt beim Blutkreislauf des Herzens. Dabei wird mithilfe der simultan erfassten Vitaldaten des Blutes und deren Auswertung über einen mit dem Bus-Sensor-System signalverbundener Filter/Prozessor die Signale der Sensoren in solche Messergebnisse aufbereitet, wie sie vom KI-System einer softwarebasierten Datenanalyse auf dem Server und von erforderlichenfalls eingebundenen Ärzten auswertbar sind. Die menschlichen physiologischen Vital-Parameter des Blutkreislaufes sind in hohem Maße voneinander abhängig und beeinflussen sich gegenseitig. Es ist daher erfindungsgemäß von großer Bedeutung, bei einer kardialen Diagnose möglichst viele Parameter der festen und flüssigen Bestandteile des Blutkreislaufes simultan zu messen, um eine effektive und objektive Diagnose zu gewährleisten.

Klassische Labor-Chemische Methoden erlauben es, die Inhaltsstoffe des Blutes sehr genau zu bestimmen, und bilden daher eine unverzichtbare Säule der medizinischen Diagnostik. Da diese Methoden ausschließlich mit dem Gang zum Arzt/Klinik/Labor und einer invasiven Blutentnahme verbunden sind und immer nur den Status quo einer einzelnen Messung zu einem bestimmten Zeitpunkt dokumentieren, sollen mit dieser Erfindung diagnoserelevante Informationen direkt beispielsweise aus dem frequenzstimulierten Infrarot- oder Raman-Spektrum verschiedener Blutmoleküle abgeleitet werden. Erfindungsgemäß wird dadurch eine simultane, permanente reagensfreie Gesundheits-Diagnostik ermöglicht, die in Verbindung mit dem Transfer über eine leistungsfähige und sichere Netzkommunikation und der KI-basierten Analyse bessere Trendentwicklungen und ein effizientes Frühwarnsystem beispielsweise bei drohenden Herzinfarkten bieten.

Welche Vitalparameter oder Biomarker des Blutkreislaufes und welche mathematischen Berechnungen und Kalkulationsverfahren dabei der Erfindung zugrunde gelegt werden, soll im Folgenden aufgrund von anerkannten und etablierten wissenschaftlichen Veröffentlichungen dokumentiert und belegt werden.

Für den Transport des Sauerstoffs $O_2$ im Blut ist ein in den Erythrozyten enthaltenes Chromoprotein-Gesamtmolekül verantwortlich, das Hämoglobin (Hb) genannt wird. Es besteht jeweils aus dem Globin und vier Häm-Molekülen. Das Globin setzt sich aus vier Untereinheiten zusammen, die jeweils ein Häm-Molekül tragen. Bei einem erwachsenen Menschen (HbA) setzt sich das Globin aus jeweils zwei $\alpha$- und zwei $\beta$-Ketten zusammen, deren Molekülmasse je ca. 16000 Dalton beträgt, so dass sich für das tetramere, also das gesamte Hb-Molekül etwa 64000 Dalton ergeben.

Bei der reversiblen Anlagerungsreaktion im Häm wird das 02-Molekül an ein zweiwertiges Eisen ($Fe_{II}$) gebunden. Diese Verbindung wird als Oxyhämoglobin (02Hb) bezeichnet, das Hb ohne gebundenen Sauerstoff heißt Desoxyhämoglobin (desoxyHb). Da sich bei der An- und Ablagerungsreaktion die Wertigkeit des Eisens nicht verändert, spricht man in der Medizin nicht von Oxidation, sondern von Oxygenation (eine Sauerstoffbindung an das zweiwertige Eisen des roten Blutfarbstoffs Hämoglobin) und umgekehrt von Desoxygenation. Die Sauerstoffkapazität des Blutes ergibt sich aus der Konzentration des $Fe_{II}$. Ein mol Hb kann an seinen vier mol Hämeisen maximal vier mol 02 binden. Berücksichtigt man die Molekülmasse, so kann 1 g Hb also 1,39 ml 02 binden. Da aber ein kleiner Teil des zirkulierenden Hb aus kaum 02-bindenden Formen wie z.B. Methämoglobin (MetHb) oder Carboxyhämoglobin (COHb) besteht, benutzt man den als Hüfner-Zahl bekannten Wert von 1,34 ml 02 pro Gramm Hb, der das Verhalten des gesamten zirkulierenden Hb exakter wiedergibt (25).

Die Menge des vom Blut aufgenommenen Sauerstoffs lässt sich über die Sauerstoffsättigung des arteriellen Blutes (Sa02) charakterisieren:

$$S_aO_2 \, (\%) \; = \; [\; cO_2Hb \, / \, (cO_2Hb \; + c_{desoxy}Hb + cMetHb + cCOHb) \,] \; x \; 100$$

Physiologisch gesehen liegen im arteriellen Blut bei allen Menschen etwa 1-2% des Hb in desoxygenierter Form vor, sowie etwa 0,5% als MetHb und 0,5-2% als COHb, was die physiologische Sa02 von etwa 96% erklärt. Da die optischen Methoden, die nur bei zwei oder drei unterschiedlichen Wellenlängen messen, diese einzelnen Bestandteile nicht exakt voneinander unterscheiden können, wird zudem eine partielle (= funktionelle) 02-Sättigung (pSa02) definiert.

Diese entspricht heute den bei markttüblichen Pulsoxymetern angegebenen Sättigungswerten (Sp02) und erfasst nur den für den Sauerstofftransport zur Verfügung stehenden Anteil des Hb:

$$pS_aO_2 \, (\%) \; = \; [\; cO_2Hb \, / \, (cO_2Hb \; + c_{desoxy}Hb \,) \,] \; x \; 100$$

Der Normalwert $pS_aO_2$ des gesunden erwachsenen Menschen beträgt ca. 98% (26).

Neben den dem Hämoglobin-Molekül zugeordneten Werten der Sauerstoffsättigung kommt ein weiterer aussagefähiger Vitaldaten-Biomarker als Hauptfaktor für gesundheitliche Risiken erfindungsgemäß zur Anwendung, der insbesondere was den Herzinfarkt und den Risikofaktor für Schlaganfall anbetrifft von herausragender Bedeutung ist, der arterielle Blutdruck (Tonus). Quellen: Kongressbericht 14.10.2012, Deutsche Gesellschaft für Kardiologie, Straus SE, Majumdar SR, Mc Alister FA. New evidence for stroke prevention. Scientific Review, JAMA 2002, 288, 1388-95

In Deutschland beispielsweise haben 6,3 Millionen eine essentielle Hypertonie, davon 3.8 Millionen eine hypertensive Herzkrankheit. Die Häufigkeit des arteriellen Bluthochdrucks nimmt auch in Abhängigkeit zum Gewichtsverhalten, sozioökonomischen Status und zum Geschlecht zu.

Quelle: Deutsche Hochdruckliga e.V. Leitlinien zur Behandlung der Arteriellen Hypertonie. www.hochdruckliga.de, 2008 9

Und 30% der Hypertoniker wissen nichts von ihrer Erkrankung und von den 50% bekannten Hypertonikern sind ca. 50% unzureichend oder nicht therapiert und gehören in die bereits einleitend erwähnte Grauzone.

Die empirisch festgelegte Klassifikation kategorisiert den Blutdruck in optimal (Diastolisch 80 mmHg, Systolisch 120 mmHg), normal (80-84, 120-129) und hochnormal (85-89,130-139) sowie in eine Hypertonie Grad I (90-99, 140-159) bis III (>189, >110) und legt außerdem eine isolierte systolische Hypertonie fest, die vor allem bei älteren Patienten vorkommt.

Die Hypertonie wird nicht als Blutdruck oberhalb eines bestimmten Grenzwertes angesehen, sondern als ein kontinuierlich ansteigender Risikofaktor verstanden, da die Beziehung zwischen Blutdruckhöhe und kardiovaskulären Erkrankungen logarithmisch-linear ist. Auch aus epidemiologischer Sicht gibt es keine offensichtliche Blutdruck - Kategorisierung, die den arteriellen Hypertonus streng gesehen definiert. Beim Erwachsenen zeigt sich sowohl für den systolischen als auch für den diastolischen Blutdruckwert ein kontinuierlicher Risikozuwachs zu kardiovaskulären Erkrankungen.

Diese kontinuierliche Beziehung zwischen arteriellem Blutdruck und kardiovaskulärem Risiko geht bis herunter zu systolischen und diastolischen Bereichen von 115 - 110 mmHg, beziehungsweise von 75 - 70 mmHg: Je niedriger der Blutdruck, desto niedriger das kardiovaskuläre Risiko.

Quelle: Mancia G, et al. 2007 Guidelines for the management of arterial hypertension. The Task Force for the Management of Arterial Hypertension of the European Society of Hypertension (ESH) and of the European Society of Cardiology (ESC), European Heart Journal 2007, 28, 1462-1536

Das Risiko für kardiovaskuläre Erkrankungen verdoppelt sich bei jedem Anstieg um 20mmHg für den systolischen Blutdruckwert, beziehungsweise bei einem Anstieg von 10mmHg für den diastolischen Blutdruckwert.2,12 Bei älteren Patienten ist das kardiovaskuläre Gesamtrisiko direkt proportional zum systolischen Blutdruck mit einem deutlich prädiktiven Wert des Pulsdrucks.

Daraus kann gefolgert werden, dass für die erfindungsgemäß angestrebte Analyse zu objektiven Diagnosestellung eines arteriellen Bluthochdruckes, der entsprechende Schwellenwert in Abhängigkeit vom kardiovaskulären Gesamtrisiko eines jeden Menschen flexibel definiert werden muss. Eine weitere Bestätigung für den Bedarf der mit dieser Erfindung zu realisierenden Aufgabe einer simultanen und kontinuierlichen Vital-Datenerfassung und Analyse mehrerer Messwerte in Form von Vitalparametern.

Denn die arterielle Hypertonie ist ein eindeutiger Risikofaktor für alle Manifestationsformen der Arteriosklerose. Der Hypertonus ist ein unabhängiger prädisponierender Faktor für Herzinsuffizienz, koronare Herzerkrankung, Apoplexie, renale Erkrankungen und Krankheiten der peripheren arteriellen Gefäße. Das Herzzeitvolumen und der peripherer Widerstand der Gefäße sind die beiden maßgebenden Faktoren des arteriellen Druckes. Die Hypertonie ist somit Folge eines erhöhten Herzzeitvolumens, eines erhöhten peripheren Widerstandes oder beider Faktoren und wird physikalisch ausgedrückt als Blutdruck = Herzzeitvolumen x Gefäßwiderstand.

Folgende Vitalparameter werden mit dem BUS-Sensor-System erfasst und erfindungsgemäß einer analytischen KI-Basierten Auswertung zu diagnostischen Zwecken verwendet:

Erstens der SVR-Wert (systemic vascular resistance) und zweitens der TPR-Wert (total pulmonal/peripheral resistance). Der TPR ist der Gefäßwiderstand, gegen den der linke Ventrikel pumpen muss. Er ist nur etwa 3 mmHg höher als der enddiastolische pulmonar-arterielle Druck.

Die Formeln lauten:

$$SVR= 80 \; x(MAP\text{-}ZVD)/HZV \quad \text{und} \quad TPR=(Pd+(PP/3))/HZV$$

Abkürzungen:

MAP: mittlerer arterieller Blutdruck (middle arterial pressure):

MAP = Diastolischer Druck + 1/3 x (Systolischer Druck - Diastolischer Druck)
ZVD: Zentraler Venendruck (Messwert)
Pd: diastolischer Blutdruck (Messwert)
PP: max. systolischer Blutdruck - minimaler diastolischer Blutdruck

Das Herzzeitvolumen auch Herzminutenvolumen genannt, ist ein Maß für die Pumpfunktion des Herzens. Das Herzminutenvolumen lässt sich durch Multiplikation des Herzschlagvolumens SV mit der Herzfrequenz berechnen. Die Herzfrequenz oder Herzschlagfrequenz ist die Anzahl der Herzschläge pro Minute. Sie wird angegeben in min$^{-1}$ oder als bpm (beats per minute).

Das Schlagvolumen SV wird meist als Differenz von enddiastolischem und endsystolischem Volumen (EDV und ESV) berechnet:

$$SV = EDV - ESV$$

Der Normalwert des Schlagvolumens beträgt circa 70 bis 100 ml. Die Schlagvolumina der rechten und linken Herzkammer sind im Wesentlichen gleich groß.

Aus dem Schlagvolumen kann man zwei weitere wichtige Funktionsparameter des Herzens ableiten: Als Ejektionsfraktion (EF) oder Auswurffraktion bezeichnet man den Anteil des vom Herzen bei einer Kontraktion ausgeworfenen Blutes, also das Schlagvolumen (SV) im Verhältnis zum Gesamtblutvolumen der Herzkammer, das Enddiastolisches Volumen (EDV) genannt wird. Sie ist ein Maß für die Herzfunktion. Das Schlagvolumen kann berechnet werden, indem man das Endsystolische Volumen (ESV) vom Enddiastolischen Volumen subtrahiert.

$$EF[\%] = \frac{SV}{EDV} * 100 = \frac{EDV - ESV}{EDV} * 100$$

Quelle: Herold, G. Innere Medizin. Eine vorlesungsorientierte Darstellung Herold, Köln, 2009

Neben den weiter oben beschriebenen Werten der Sauerstoffsättigung und den dargestellten Blutdruck-Vitalparametern kommen weitere aussagefähige Vitaldaten-Biomarker im Molekülbereich erfindungsgemäß zur Anwendung, die nach dem aktuellen Standard der Herzinfarkt-Diagnose von ausschlaggebender Bedeutung sind und frequenzstimuliert über IR (Infrarote Lichtwellenlänge) oder NIR-Spektroskopische (Nahinfrarot) ermittelt werden.

Die Wechselwirkung von elektromagnetischer Strahlung mit den Dipolmomenten von Molekülen ermöglicht im Zusammenhang mit spezifischen Molekülschwingungen erfindungsgemäß eine erweiterte Analyse und Auswertung von zusätzlichen BioMarkern, die bisher nur laborchemisch möglich waren. Zum einen existiert ein bekanntes Resonanzverhalten von Molekülen für die Wechselwirkung des permanenten elektrischen Dipolmoments mit elektromagnetischer Strahlung derjenigen Frequenz, bei der ein Molekül seine Eigenschwingungen ausführt. Diese Strahlung liegt typischerweise im mittleren Infrarot (MIR), also bei Wellenlängen zwischen 3 und 50 $\mu$m. Zum anderen kann die Einstrahlung auf ein Molekül ein molekulares Dipolmoment induzieren. Dieses Phänomen liefert spektral messbare Beiträge in Form einer Rayleigh-Streuung der eingestrahlten Frequenz, sowie zusätzliche Anteile bei einer um die molekulare Schwingungsfrequenz erhöhten oder erniedrigten Frequenz. Diese sogenannte inelastische Raman-Streuung tritt in der Regel mit Licht im sichtbaren, nahe ultravioletten oder nahen infraroten Spektralbereich auf.

Quelle: P.Lasch, J. Kneipp, Biomedical Vibrational Spectroscopy, JohnWiley&Sons, New York 2008

Sowohl die MIR- als auch die Raman-Spektroskopie haben ihre spezifischen Vor- und Nachteile. Im Gegensatz zu gasförmigen Proben wie der Atemluft spielt bei wässrigen Proben - und Blut besteht zu einem wesentlichen Teil aus Wasser - die Absorption von Wasser eine entscheidende Rolle. Auch Wasser besitzt die Grundmoden seiner Schwingungen bei Frequenzen im Infraroten. Die resonante Wechselwirkung eines molekularen Dipolmoments mit elektromagnetischer Strahlung birgt daher im Hinblick auf das erfindungsgemäß angestrebte Analyseverfahren biologischer Vitaldaten-Proben ein sehr gutes Signal-zu-Rausch-Verhältnis der Mittelinfrarotspektroskopie. Leider wird das Signal jedoch von den alles überlagernden Wasserbanden beeinflusst. Eine 0,1 mm dicke Wasserschicht absorbiert eine durchgehende MIR-Strahlung der Wellenlänge 10 $\mu$m zu fast 99,9 %.

Anders ist die Situation im sogenannten biologischen Fenster der sichtbaren Strahlung und angrenzender Frequenzbereiche. Hier absorbiert Wasser praktisch nicht, sodass das typischerweise zur Raman-Spektroskopie verwendete Licht sehr gut die wässrige Probe durchdringt und das inelastisch gestreute Licht die molekularen Schwingungen der Inhaltsstoffe offen legen kann. Der Wirkungsquerschnitt der Raman-Streuung ist allerdings sehr klein, sodass die Raman-Spektroskopie in der biomedizinischen Optik gegenüber der MIR-Spektroskopie bisher keine praktische Anwendung gefunden hat und zudem führt die Bestrahlung biologischer Substanzen mit sichtbarem oder UV-Licht häufig zu Fluoreszenzlicht, das dem relativ kleinen Raman-Signal als Untergrund ein beträchtliches Rauschen überlagern kann. Nahinfrarote Strahlung verursacht weniger Fluoreszenz, führt aber rasch zu sehr kleinen Signalen, da der Raman-Wirkungsquerschnitt überproportional stark von der Wellenlänge abhängt.

Mit dieser Erfindung und den folgenden Berechnungen wird gezeigt, wie diese Aufgabe einer praktikablen Lösung zugeführt werden soll.

Ein zweiatomiges Molekül mit dem permanenten elektrischen Dipolmoment $d_o$ und der Polarisierbarkeit $\alpha$ sei einem äußeren elektrischen Feld E ausgesetzt. Für kleine Auslenkungen des Abstandes R zwischen den beiden Atom-kernen um die Ruhelage $R_o$ lassen sich $d_o$ und $\alpha$ nähern durch

$$d_0 \approx d_0(R_0) + \left.\frac{\partial d_0}{\partial R}\right|_{R_0} (R - R_0) + \ldots$$

und

$$\alpha \approx \alpha(R_0) + \left.\frac{\partial \alpha}{\partial R}\right|_{R_0} (R - R_0) + \ldots$$

Schwingt das Molekül mit der Kreisfrequenz $\omega$ um seine Ruhelage, so gilt in harmonischer Näherung

$$R(t) - R_o = r \cdot cos\ (\omega, t).$$

Für das elektrische Feld mit der Schwingungsfrequenz $\omega$ gilt $E(t) = E \cdot cos(\omega t)$.

Somit resultiert für das Gesamtdipolmoment

$$d = d_0 + \alpha E \approx d_0(R_0) + \frac{\partial d_0}{\partial R}\bigg|_{R_0} r \cos(\omega_v t) + \alpha(R_0) E_0 \cos(\omega t) + \frac{\partial \alpha}{\partial R}\bigg|_{R_0} r \cos(\omega_v t) E_0 \cos(\omega t) + \dots$$

Mit *2cos x cos y = cos (x + y) + cos (x - y)* folgt hieraus eine Summe von fünf Termen:

❖ das konstante elektrische Dipolmoment $d_0 (R_0)$

❖ die für die Infrarotspektroskopie relevante dynamische Änderung des permanenten Dipolmoments bei der Eigenfrequenz der Molekularschwingung

$$r \frac{\partial d_0}{\partial R}\bigg|_{R_0} \cos(\omega_v t)$$

❖ die als Rayleigh-Streuung bezeichnete elastische Streuung der einfallenden Strahlung $\alpha \cdot R_0 \cdot E_0 \cdot cos(\omega t)$

❖ der Teil der Raman-Streuung, bei dem das gestreute Licht eine um die Schwingungsfrequenz erniedrigte Frequenz aufweist (Stokes-verschoben)

$$\frac{rE_0}{2} \frac{\partial \alpha}{\partial R}\bigg|_{R_0} \cos((\omega - \omega_v)t)$$

❖ und der Teil der Raman-Streuung, bei dem das gestreute Licht eine um die Schwingungsfrequenz erhöhte Frequenz aufweist (Anti-Stokes-verschoben)

$$\frac{rE_0}{2} \frac{\partial \alpha}{\partial R}\bigg|_{R_0} \cos((\omega + \omega_v)t)$$

Die Infrarotspektren treten somit bei der Frequenz der Eigenschwingung des Moleküls auf und basieren auf Änderungen des permanenten Dipolmoments während einer Molekülschwingung.

Mit $\omega \gg \omega_v$ liegen die Raman-Spektren hingegen meist im sichtbaren und unmittelbar angrenzenden IR-Spektralbereich. Ihre Amplitude ergibt sich aus der Änderung der Polarisierbarkeit während der Schwingung.

Für die erwähnte MIR-Spektroskopie gibt es deshalb erfindungsgemäß die Überlegung, den Einfluss der starken Wasserabsorption mit einer intensiven Lichtquelle in Form einer Quantenkaskadenlaser-Diode zu reduzieren und ein besseres Signal-Rauschverhältnis zu erreichen. Dadurch ist es für einige wichtige Molekül-Inhaltsstoffe der Hautschichten und des Blutes möglich, mit bestimmten Auswertalgorithmen unabhängig von der Spektroskopie-Methode die Konzentration mehrerer Inhaltsstoffe simultan zu bestimmen. Lediglich der Auswertalgorithmus im KI-basierten Service-Zentrum beim Server legt fest, welche Kombination von Schwingungsbanden für welche Konzentrationsbestimmung der Moleküle herangezogen wird. Damit wird es erfindungsgemäß ermöglicht, auf der Basis von Mustererkennungsverfahren anhand der gemessenen und erfassten Substanz-, Blut- und Molekül-Spektren die frequenzstimulierte Schwingungsspektroskopie für eine vollkommen neue KI-basierte Analyse und objektivierte Diagnose zur Früherkennung von gesundheitlichen Problemen einzusetzen.

Wie gerade dargestellt, reflektieren, transferieren oder absorbieren alle Atome und Moleküle ganz charakteristische, für das Molekül spezifische Wellenlängen des Lichtspektrums. Diese Eigenschaft soll in dieser Erfindung genutzt werden um die Konzentrationen chemischer Stoffe in Gasen oder Flüssigkeiten zu bestimmen.

Wird dieses Verfahren für den Nachweis von 02Hb und desoxyHb benutzt, so spricht man hier ebenfalls von Oxymetrie. So wie alle Proteine ändert auch das Hb seine räumliche Konfiguration, wenn es an chemischen Reaktionen teilnimmt. Jede dieser Konfigurationen besitzt ein eigenes Lichtreflexions- bzw. -absorptionsmuster, das sich die unterschiedlichen Verfahren der Pulsoxymetrie zu Nutze machen. Die Transmissionspulsoxymetrie mit Fingerclip-Sensoren, bei der Licht von einer Leuchtdiode (LED) durch das Gewebe gestrahlt und auf der gegenüberliegenden Seite von einer Photodiode detektiert wird, ist heute das Standardverfahren in der Medizin.

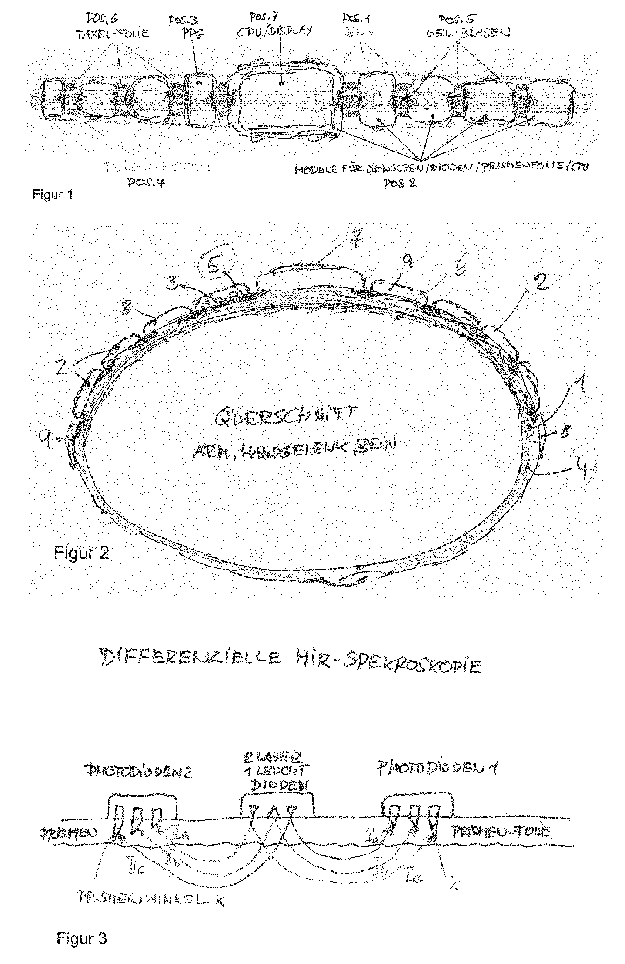

Sie basiert auf den unterschiedlichen Absorptionsspektren (s. Abb.1 unter Abb1-4.pdf) von oxygeniertem (02Hb) und desoxygeniertem Hämoglobin (desoxyHb). Gemessen wird jeweils bei zwei unterschiedlichen Wellenlängen; zum einen bei 660 nm im Bereich des roten Lichts, da das desoxyHb hier am meisten Licht absorbiert und die Extinktionen von 02Hb und desoxyHb die größte Differenz aufweisen, zum anderen bei 940 nm im infraroten Bereich, da hier das 02Hb ein Absorptionsmaximum erreicht. Die Messung bei diesen beiden Wellenlängen ermöglicht die bestmögliche Unterscheidung zwischen oxygeniertem und desoxygeniertem Hämoglobin.

*Siehe Absorptionsspektren Abb.1 von oxygeniertem und desoxygeniertem Hämoglobin, Myoglobin und Gesamttroponin unter Anlage 1, Abb1-4.pdf.*

Ein weiteres Verfahren, das bisher allerdings noch keinen routinemäßigen Einsatz in der medizinischen Anwendung findet, ist die Reflexionspulsoxymetrie. Auch hierbei wird Licht von einer Leuchtdiode in das Gewebe hineingestrahlt. Die Photodiode liegt aber in unmittelbarer Nähe der Leuchtdiode LED auf derselben Oberfläche des Gewebes auf und misst das vom Gewebe reflektierte Licht. Dieses Verfahren beruht auf den unterschiedlichen Reflexionsspektren (Abb.2 unter Abb1-4.pdf) von 02Hb und desoxyHb. Auch bei der Reflexionspulsoxymetrie wird bei 660 nm im roten Bereich gemessen, da hier die Differenz der Reflexionsspektren von oxygeniertem und desoxygeniertem Hämoglobin am größten ist. Da das eigentlich zu bestimmende 02Hb hier bereits sein Reflexionsmaximum aufweist, wird als Referenz im infraroten Bereich an dem Punkt gemessen, an dem 02Hb und desoxyHb gleich viel Licht reflektieren. Dieser Punkt liegt bei 890 nm.

*Siehe Reflexionsspektren Abb. 2 von oxygeniertem und desoxygeniertem Hämoglobin, Gesamt-Troponin und Myoglobin unter Anlage 1, Abb1-4.pdf.*

Nach dem Lambert-Beer-Gesetz kann die Konzentration eines Stoffes, z.B. des oxygeniertem Hb, berechnet werden. Es besagt, dass die Extinktion (E) einer Lösung direkt proportional zur Konzentration (c) der darin gelösten lichtabsorbierenden Stoffe ist, wenn ihr molekularer Extinktionskoeffizient ($\varepsilon$) und die optische Wegstrecke (d) bekannt und konstant sind gilt:

$$\mathbf{E} = \mathbf{\varepsilon \cdot c \cdot d}$$

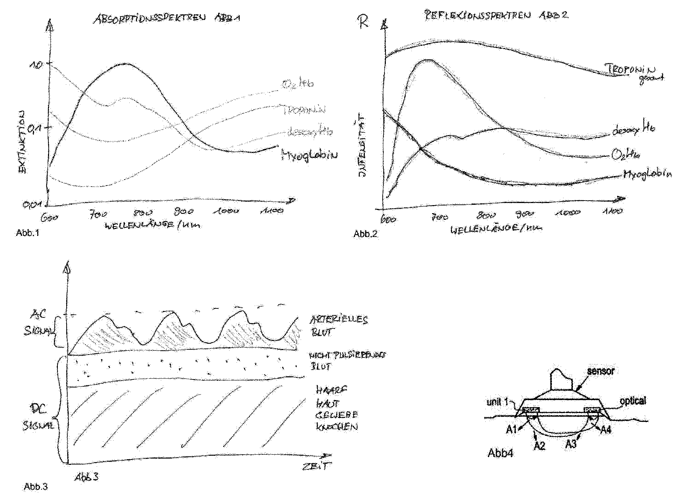

Da sich durch diese Konzentrationsbestimmung alleine jedoch nicht auf die tatsächliche Konzentration des oxygenierten Hb im arteriellen Blut schließen lässt, messen alle Pulsoxymeter nach folgendem Prinzip (Abb.3 unter Abb1-4.pdf), um arterielles und venöses Blut besser differenzieren zu können. Die arterielle Pulsation ruft eine zeitliche Änderung der Lichtabsorption hervor, die bei beiden Messwellenlängen getrennt photometrisch gemessen wird. Diese Schwankungen werden als AC-Signal ("alternate current") bezeichnet. Dem gegenüber steht eine nahezu konstante Lichtabsorption bzw. -reflexion über die Zeit, die kontinuierlich mitgemessen wird. Sie entsteht durch nicht-pulsatiles arterielles und venöses Blut, sowie durch Haare, Haut, Gewebe und Knochen. Die Gesamtheit dieser statischen Signale bildet das sog. DC-Signal ("direct current").

Siehe Abb. 3 in der Anlage Abb1-4.pdf, Quelle: Alexander CM, Teller LE, Gross JB. Principles of pulse oximetry: theoretical and practical considerations. Anesth Analg 68 (3): 368-76, 1989.

Aus den genannten Lichtsignalen lässt sich die Messvariable Omega ($\Omega$) - teilweise auch als R bezeichnet, die auf der Grundlage des Lambert-Beer-Gesetzes mit der Sauerstoffsättigung theoretisch in einer nahezu linearen Beziehung steht, wie folgt errechnen:

$$\Omega = \frac{\left(\frac{AC}{DC}\right)_{rot}}{\left(\frac{AC}{DC}\right)_{infrarot}}$$

Quelle: Schmitt JM. Simple photon diffusion analysis of the effects of multiple scattering on pulse oximetry. IEEE Trans Biomed Eng 38 (12): 1194-203, 1991.

Als Ursache für das Abweichen der empirischen Beziehungslinie von $S_pO_2$ und $\Omega$ von der theoretischen Berechnung und den daraus resultierenden Messfehler im niedrigeren Sättigungsbereich wird das DC-Signal angesehen. Neben den weitestgehend konstant bleibenden Faktoren Haut, Gewebe und Knochen kommt hier vor allem der inkonstante Parameter der Perfusion des Gewebes in Betracht.

Um die Auswirkungen dieser Störeinflüsse zu verringern und somit die Messgenauigkeit erheblich zu verbessern, wurde von Bernreuter eine neue Sensortechnologie entwickelt: Siehe Abb. 4 unter Anlage Abb1-4.pdf, Reflexoxymetrie Sensor nach Bernreuter, Patent-Nr.: US 6.226.540.

Neu ist bei diesem - nach dem Prinzip der Reflexionspulsoxymetrie messenden - Sensor nicht nur eine Modifikation der Messwellenlänge im infraroten Bereich (910 nm statt 890 nm), sondern vor allem die Anzahl und die Anordnung der Licht- und Photodioden. An der Auflagefläche des Sensors sind zwei aus licht emittierenden Dioden (LED) und Photodioden kombinierte Sensoren-Paare spiegelbildlich zueinander angebracht. So ergeben sich, im Gegensatz zu herkömmlichen Sensoren, die jeweils nur über einen Sender und einen Empfänger verfügen, zusätzlich zu den beiden langen Lichtwegen durch das Gewebe (A2 + A3) zwei sehr kurze Lichtwege (A1 + A4) und somit auch die vierfache Anzahl der pro Messung registrierten $\Omega$-Werte ($\Omega$1-4).

Neben der Registrierung von $\Omega$1-4 wurden aus diesen noch die Werte $\Omega_{mean}$ und $\Omega_{diff}$ errechnet, wobei $\Omega_{mean}$ der Mittelwert der jeweiligen $\Omega$1-4 ist

$$\Omega_{mean} = \frac{\Omega_1 + \Omega_2 + \Omega_3 + \Omega_4}{4}$$

und $\Omega_{diff}$ der Mittelwert aus den letzten Einhundert korrelierten Messwerten (f) der entsprechenden Lichtwege ($\Omega_c$), der somit auch die Lichtabschwächung berücksichtigt:

$$\Omega_{diff} = \frac{f(\Omega_{1c}) \cdot \Omega_1 + f(\Omega_{2c}) \cdot \Omega_2 + f(\Omega_{3c}) \cdot \Omega_3 + f(\Omega_{4c}) \cdot \Omega_4}{4}$$

Bei erhöhter Absorption werden Lichtanteile, die längere Strecken durch das Gewebe zurücklegen, verhältnismäßig stärker abgeschwächt als diejenigen, die kürzere Strecken durchqueren. Variiert der Blutgehalt im Gewebe, so ist ähnliches zu beobachten. Dies führt dazu, dass mit abnehmendem Blutgehalt des Gewebes die mittlere und differentielle Wegstrecke, die die im Pulsoxymeter registrierten Photonen zurückgelegt haben, verlängert wird.

Daraus ergibt sich, dass die gemessene $S_pO_2$ nicht nur von der Messvariablen $\Omega$, sondern auch von anderen Variablen, z.B. der Perfusion, abhängt. Um die gesamte Lichtabsorption (LA), die näherungsweise dem Logarithmus des DC-Signals entspricht, zu bestimmen und somit die Störeinflüsse in der Berechnung zu eliminieren, kann man folgendermaßen vorgehen:

$$\ln DC \approx LA = \frac{\overbrace{(A2 + A3)}^{\substack{\text{lange} \\ \text{Lichtwege}}} - \overbrace{(A1 + A4)}^{\substack{\text{kurze} \\ \text{Lichtwege}}}}{2}$$

*Berechnung der Störeinflüsse durch Berechnung der Lichtabschwächung Patent-Nr.: US 5,922,607*

Subtrahiert man also die Summe der Absorptionen der beiden kurzen Lichtwege (A1 + A4) von der Summe der Absorptionen der beiden langen (A2 + A3), so erhält man die doppelte LA des Gewebes, die - dividiert durch 2 - näherungsweise dem natürlichen Logarithmus des DC-Signals entspricht. Durch die Differenzbildung der Lichtabsorptionen gehen die Lichtintensitäten der beiden Licht emittierenden Dioden deutlich geringer in die resultierende Lichtabschwächung ein und auch die optischen Eigenschaften der Hautoberfläche (Behaarung, Pigmentierung) beeinflussen das Messergebnis wesentlich weniger. Diese Erkenntnisse und die von Bernreuter vorgeschlagenen Berechnungs- und Verfahrensprozeduren werden bei der Ermittlung der Vitaldaten-Parameter durch das Bus-Sensor-System und bei deren Auswertung zur Berechnung der Analyse- und Diagnose-Daten erfindungsgemäß in erweiterter Form einfließen und durch ein erweitertes Sensor/Diodensystem mit Microprismen nach Figur 3 ein wesentlich sichereres differentielles Mess- und Auswertverfahren ermöglichen.

Diese Messeinheit des Bus-Sensor-Systems für die Mittlere IR-Spektroskopie (MIR) oder die NahInfrarot-Spektroskopie (NIR), wie es erfindungsgemäß modifiziert zur Anwendung kommen soll, beinhaltet wenigstens ein Laser-System der Klasse I, das aus mehreren Laserdioden besteht, die frequenzstimuliertes nahinfrarotes Licht verschiedener der Wellenlängen wie etwa 775, 810, 850 und 910nm bei einer Spektralbreite von 5nm generieren. Das emittierte Licht wird nach Durchgang durch das Gewebe von mehreren aktiven und passiven Filtern, Miniaturprismen und schließlich durch spezielle Photodioden detektiert, in elektronische Impulse konvertiert, aufbereitet, verstärkt und zwischengespeichert. Mit der Transformation zum Server der zentralen Recheneinheit erfolgt die Auswertung sowie die Bewertung und ggf. eine optische Darstellung der Messdaten. Figur 3 zeigt eine schematische Darstellung des modifizierten Messprinzips im Bus-Sensor-System.

*Siehe Figur 3 Messeinheit der differenziellen frequenzstimulierten MIR-Spektroskopie*

Die Darstellung nach Figur 3 zeigt das Laser- und Weisslicht-Diodensystem, die dreigeteilte Photodiode, die zur Detektion von sechs unterschiedlichen Lichtwegen (la, Ib, Ic, IIa, IIb und IIc) durch das Gewebe über Micro-Prismen führt, die in einer transparenten Folie eingebettete sind. Der Steigungswinkel der Primen (k) geht dabei in die Berechnung des Gewebe-Oxygenierungsindex (tissue oxigenation index, TOI) mit ein.

Bei den durch die differenzielle MIR- Spektroskopie bestimmten Messparametern des darunterliegenden Gewebes unterscheidet man zwischen direkt gemessenen, errechneten und abgeleiteten Vital-Parametern, sowie zusätzlich die Konzentrationen mehrerer Inhaltsstoffe. Dabei bestimmt der Auswertalgorithmus im KI-basierten Service-Zentrum beim Server, welche Kombination von Schwingungsbanden für welche Konzentrationsbestimmung der Moleküle herangezogen wird. Damit wird es erfindungsgemäß ermöglicht, auf der Basis von Mustererkennungsverfahren anhand der gemessenen und erfassten Substanz-, Blut- und Molekül-Spektren die frequenzstimulierte Schwingungsspektroskopie für eine vollkommen neue KI-basierte Analyse und objektivierte Diagnose zur Früherkennung von gesundheitlichen Problemen einzusetzen.

Vom Bus-Sensor-Systems direkt gemessen werden zunächst mit der MIRS Änderungen der Konzentration beispielsweise des oxygenierten Hämoglobins und des desoxygenierten (reduzierten) Hämoglobins. Dabei handelt es sich nicht um Absolutwerte, sondern vielmehr um Konzentrations-änderungen von einem Ausgangsniveau, d.h. es wird die Änderung der Molarität ($\Delta\mu$mol/ml) angegeben. Die gemessenen Änderungen der Konzentrationen von 02Hb und des rHb spiegeln die regionale Durchblutung, das Sauerstoffangebot und die Sauerstoffausschöpfung des Gewebes wider. Beide Parameter bilden die Grundlage zur Errechnung der Änderung des Gesamthämoglobins ($\Delta$cHb), des Hämoglobinindex (tissue hemoglobine index, THI) und des Gewebeoxygenierungsindex (tissue oxygenation index, TOI). Dabei entspricht die Summe aus $\Delta$O2Hb und $\Delta$rHb dem Gesamthämoglobin respektive seiner Konzentrationsänderung ($\Delta$cHb). Die Differenz aus $\Delta$O2Hb und $\Delta$rHb führt zur so genannten Hämoglobindifferenz, die zur Beschreibung der Veränderung der zerebralen Oxygenierung herangezogen werden kann.

$$\textbf{TOI} = \frac{\textbf{k} \cdot \textbf{O}_2\textbf{Hb}}{\textbf{k} \cdot \textbf{O}_2\textbf{Hb} + \textbf{k} \cdot \textbf{rHb}}$$

Dieser Gewebeoxygenierungsindex (TOI), entspricht in gewissen Grenzen auch der Änderungen der Sauerstoffsättigung im Hautgewebe von einer gedachten Grundlinie und spiegelt Änderungen des Blutflusses mit höherer Sensitivität wider. Die Streuung des Lichts im Gewebe geht dabei durch Anwendung des von Suzuki beschriebenen Prinzips der "spatial resolved spectroscopy" in die Berechnung mit ein.

Quelle: Suzuki S, Takasaki S, Ozaki T, Kobayashi Y. Tissue oxygenation monitor using NIR spatially resolved spectroscopy In: Proc. SPIE, 04/1999, 1999

Um den Herzgesundheitszustand über das Blut zu untersuchen sind in der klassischen kardiovaskulären Diagnostik eine Vielzahl von medizinisch relevanten Biomarkern definiert worden, die erfindungsgemäß auch in dieser Projektentwicklung zur Anwendung kommen sollen.

Nicht nur EKG, Herzfrequenz und Sauerstoffsättigung sind dabei von Bedeutung, sondern auch wie oben gezeigt, die Messung des Blutdrucks, insbesondere aber eine Bestimmung der Blut- und Gewebeinhaltstoffe, wie Troponin I, Myoglobin, CK-MB und/oder IMA (Ischemia Modifed Albumin). Dabei kommen bekannte Verfahren der differenziellen NIR- und MIR-Spektroskopie zum Einsatz, indem Absorptions-, Reflexions, und Transmissionsmessungen mit sichtbarem bzw. nahinfrarotem Licht und/oder Laserdioden durchgeführt werden. Eine große Gewichtung erlangten in letzter Zeit die kardialen Troponine, nicht nur wegen ihrer außerordentlichen Kardiospezifität, sondern auch wegen ihrer hervorragenden Sensitivität mit Bezug auf den Herzinfarkt. Daher wurden andere Biomarker wie die Kreatinkinase weitgehend abgelöst und Troponine spielen die zentrale Rolle in der kardialen Diagnostik. Im Vordergrund derartiger auf das Herz bezogener Messungen mit dem Bus-Sensor-System und Verfahren stehen zunächst die Überwachung der fraktionellen Sauerstoff-Sättigung, der Hämoglobinkonzentrationen, sowie der Herzfrequenz, des Blutdrucks, der Temperatur und der Herzfrequenzvariabilität und des Einkanal-EKG's zur Detektion von Standardabweichungen im Vergleich mit auf dem Server vorhandenen Standard- und Vorlaufwerten.

Ergibt sich hierbei eine Abweichung von den kardiovaskulären Standardwerten kommen Laser-Spektroskopische Methoden zur Ermittlung infarktspezifischen Parameter wie Troponin I, Myoglobin und IMA durch Verfahren ähnlich der optischen Kohärenztomographie zur Anwendung und das Notfallsystem wird eingeleitet. Die Messungen sind aufgrund der optischen Sensoren nichtinvasiv und können für längere Zeit ohne Nebenwirkungen zur Überwachung eingesetzt werden.

Stand der Technik

Zahlreiche derzeit am Markt für gesundheitliche Vorsorge und Notfälle zur Verfügung stehenden persönlichen Hilfsmittel wie z. B. Mobiltelefone, HomeSense, SensSave (Fraunhofer Gesellschaft), Sicherheitssysteme im Hause, Nachttisch-Telefone, Automobil-Assistenzsysteme leisten nur Hilfestellung, wenn dem Betroffenen bewusst wird, was gerade passiert und er auch noch bei Bewusstsein ist. Keines dieser verfügbaren Produkte/Dienste stellt nämlich auch nur eine eingeschränkte automatische Erkennung, Analyse und Bewertung einer herzgesundheitlichen Notfallsituation zur Verfügung. Im Notfall kann man zudem nur dann mit einem Handy einen Arzt oder Krankenwagen rufen, wenn man noch handlungsfähig und bei vollem Bewusstsein ist. Fällt man und bricht sich beide Hände/Arme oder wird man im Wintersport unter einer Lawine begraben, kann man wohl kaum noch telefonieren und einen Retter rufen, wenn man bewegungs-eingeschränkt oder unter Sauerstoffmangel bewusstlos wird. Darüber hinaus ist jedes derzeit am Markt vorhandene System oder Produkt durch seine durchaus multifunktionalen aber überwiegend eindimensionalen Anwendungen gekennzeichnet.

Im Allgemeinen sind die vorhandene Produkte und Dienste so konzipiert, indem sie nur eine bestimmte vorgegebene Art von gesundheitlichen Service- und Notfall-Situationen lösen können. Für unbewusste Situationen eines Notfalls der Herzgesundheit, die nicht klar erkannt und zugeordnet werden können, gibt es aber weitestgehend kein Produkt und keine Dienstleistung mit automatischer Analyse und Bewertung durch ein simultanes und permanentes Bus-Sensor-System mit KI-basierter automatischer Analyse. Darüber hinaus haben die meisten Produkte und Dienste für gesundheitliche Betreuung und Notfälle andere inhärente Beschränkungen durch örtliche Gegebenheiten, regionale Vorschriften und/oder landespolitische Gesetzgebung mit oftmals zusätzlichen wirtschafts- und gesellschaftspolitisch einschränkenden Faktoren.

Was fehlt, ist ein universelles und weltweit einsetzbares Frühwarn- und Vorsorgesystem mit individueller sensorischer Erfassung zum Beispiel der wichtigen Herz-Vitalparameter eines Menschen nach einem internationalen Standard, das ebenso Vitalparameter über die Stress- und Belastungssituation des Körpers einschließt. Mehrere Faktoren deuten darauf hin, dass jetzt der richtige Zeitpunkt für eine solches universelles Herzgesundheits-Frühwarn- und Notfallsystem gegeben ist:

1. Die Nutzung der internationalen Kommunikation über Satellit und Mobilfunknetze mit erst seit kurzer Zeit möglichen hohen Datentransferleistungen erfährt verbunden mit leistungsfähigeren Endgeräten wie Smartphones und Tablet-PC einen zunehmenden Einsatz bei breiten Bevölkerungsschichten.

2. Die Kosten für eine derartige internationale Datenkommunikation, wie sie im Rahmen dieser Erfindung erforderlich wird, sind in den letzten Jahren durch die Massenanwendung ganz erheblich reduziert worden.

3. Die moderne Datenkompression und Verschlüsselung wie sie beispielsweise Afaria von SAP oder envoy von Saltation u.a. bieten, garantiert nach dem internationalen Standard ISO/IEC 15408 schnellen und sicheren Datentransfer insbesondere für personenbezogene vertrauliche Daten erst seit wenigen Monaten.

4. Moderne Softwareanwendungen und -Methoden der Massendaten-verarbeitung und Datamining ermöglichen mit KI-Systemen die automatischen Analysen, Auswertung und Verarbeitung großer Datenmengen, wie sie erfindungsgemäß mit den Herz-Vitalparametern anfallen.

5. Die erfindungsgemäß erforderliche automatische KI-Datenverarbeitung großer Datenmengen ist mit den Entwicklungen der In-Memory-Management-Prozessdatenverarbeitung, wie sie HANA von SAP oder "big-data" von SiSense's bieten, überhaupt erst seit etwa 1-2 Jahren möglich.

6. In breiten Bevölkerungsschichten, insbesondere bei der zunehmenden älteren Generation, steigt das Bewusstsein, mit modernen Methoden der Früherkennung von gesundheitlichen Schwachstellen rechtzeitig vorbeugende Maßnahmen zu ergreifen.

7. Betriebliche Gesundheitsvorsorge gewinnt zunehmend unter den Entscheidungs- und Verantwortungsträgern in Wirtschaft und Verwaltung an Bedeutung.

8. Die Themen Quantifiing Self und Self Qualify, d.h. die Erfassung persönlicher Bewegungs- und Verhaltensdaten mit Sensoren am Körper und deren Offline-Auswertung und Analyse zur Bewertung und Steigerung der eigenen Leistungsfähigkeit und Belastbarkeit gewinnt steigendes Interesse.

Damit wird deutlich, dass es zukünftig einen erweiterten Bedarf an speziellen gesundheitlichen Frühwarn- und Notfallsystemen geben wird, der erfindungsgemäß auch mit dem Schwerpunkt der Herzgesundheit verbunden werden kann und universell für eine Vielzahl von verschiedenen persönlichen, organisatorischen und betrieblichen Bedürfnissen allein bei einem gesunden Menschen zutreffend und nützlich sein wird.

Betrachten man zudem allgemein das Problem unseres Gesundheitssystems mit mehr oder weniger diskontinuierlichen oder sporadischen Gesundheitsdienstleistungen bei Personen mit chronischen Erkrankungen, dann ergibt sich ein weiteres erfindungsgemäß sinnvolles Anwendungspotenzial, das in einem erheblichen Umfang zur Kostenreduzierung beitragen kann, wenn zukünftig periodisch aber stetig simultan erfasste Vital-Daten über die gesundheitliche Entwicklung eines Menschen zunächst anonym und verschlüsselt dokumentiert werden und bei Bedarf im Notfall zur Verfügung stehen könnten. Die Sicherheit und Verlässlichkeit einer Gesundheits-Analyse und -Diagnose wären im Vergleich zum heutigen Verfahren um ein Vielfaches sicherer und unabhängig von den persönlichen Einschätzungen eines einzelnen Arztes.

Allein in den USA sterben jedes Jahr etwa 80.000 Menschen aufgrund von Fehldiagnosen. Ärzte diagnostizieren eine Erkrankung oftmals unbewusst nur deshalb, weil sie mit den ihnen bekannten Methoden erfolgreich zu behandeln wäre.

Quelle: Spiegel Nr.7 vom 14.2.2011

Bemühungen in Deutschland die Gesundheitsvorsorge und die Diagnosen zu verbessern, sind von der Anzahl und der Arzt- oder Klinikbesuche bestimmt und abhängig von den dabei gelegentlich gesammelten und manchmal nur regional gespeicherten Informationen und Daten. Das kurzperiodische aber kontinuierliche und simultane Erfassen und Speichern von biometrischen HerzGesundheits- und Lebens-Vitaldaten des menschlichen Körpers ist derzeit nur eingeschränkt auf Spezialfälle der Intensiv-Versorgung und -pflege möglich und wenn, dann nur mit einem hohen Kosten-, Material- und Geräteaufwand. Dies erzeugt eine Situation, in der zum Beispiel die Wirksamkeit und die Nebenwirkungen eines bestimmten Medikamentes bei der Behandlung einer bestimmten Krankheit nur punktuell oder lokal durch eine klinische Studie mehr oder weniger zuverlässig festgestellt werden kann.

Erfindungsgemäß ist durch die konzipierte Bus-Sensor-System-Integration die Messung und Erfassung der Vitaldaten nur an einer Stelle des Körpers aber variabel, abhängig von der Anwendung und auf die Extremitäten bezogen erforderlich. Figur 1 zeigt eine schematische Draufsicht auf das System, wobei die einzelnen Module nicht sichtbar in einem flexiblen und/oder halbstarren Gesamtträger wahlweise integrierbar sind. Figir 2 zeigt eine Seitenansicht als Querschnitt. Das als Multisensor ausgestaltete Bus-Sensor-System nutz erfindungsgemäß eine Mehrzahl von Sensoren in modulartiger Ausgestaltung wie in den Figuren 1 und 2 mit der Pos. 2 gekennzeichnet über eine Bus-Band-Vorrichtung entsprechend Pos. 1, (rote Linien), so dass nicht an verschiedene Körperstellen mit jeweils einem separaten Mess-System/-Gerät gemessen werden muss.

Die Module Pos. 2 zur Erfassung von Messwerten können runde, rechteckige oder quadratische Formen haben. Das simultane Monitoring mehrerer Umgebungsparameter, das z.B. die Körpertemperatur, die Bewegung, den Luftdruck und Höhenwerte parallel zu anderen Vital-Werten kontinuierlich erfasst und dem Bus-Sensor-System als Parameter zugeführt wird, ermöglicht eine bessere Einschätzung und Zuordnung zu bestimmten Befindlichkeiten der Person mit Bezug auf die Gesamtheit der ermittelten Vitalparameter, so dass auch diesbezüglich und hinsichtlich davon abhängiger Parameter eine aussagefähigere Vitaldatenerfassung stattfinden kann.

Analog wird mittels eines speziellen Moduls und in Kombination nach Pos. 5 wahlweise über den Bus des Bus-Sensor-Systems mindestens ein mechanischer Drucksensors in Gel-Blasenform ausgestaltet integriert, der mit einer Taxel-Folie Pos. 6 (blau Linie) ausgerüstet ist und die gesamte Kontaktstruktur des Bus-Sensor-System-Bandes erfasst, um Artefakte zu kompensieren, die aus der Bewegung das Sensorträgers resultieren können.

Quelle: W. Wu, Science Express, 15.4.3013, DOI:10.11261 science 1234885, Physik Journal, 12. Jahrgang, Juni 2013, S. 14.

Die rückwärtige, am Köper anliegende Struktur des Bus-Sensor-Systems ermöglicht mit den kleinen gelgefüllte Blasen bzw. Kissenstrukturen Pos 5, dass ein dichtes Anliegen der Folie mit den integrierten optischen Mess-Systemen sichergestellt wird und die erforderlichen Kontakte zur Hautoberfläche über Pos 6, Taxel-Folie, blaue Linie vorliegen. Hier sind zudem erweiterte Drucksensoren integriert, die den Anpressdruck der Messfolie erfassen und in die Auswertung einfließen lassen können.

Um für den Träger des Bus-Sensor-Systems wahlweise am Handgelenkt getragen eine Einsicht und Übersicht der Messdaten visuell durchführen zu können ist es vorteilhaft, wenn die Messwerte nicht nur ermittelt, sondern auch weiterverarbeitet und/oder zur Inaugenscheinnahme über ein kleines Display-Modul mit integrierter CPU ausgegeben werden, Pos. 7; hierin kann zusätzlich eine Uhr für die Anzeige der Tageszeit ausgestaltet sein. Ein wichtiger Faktor bei der Genauigkeit des Detektionsverfahrens ist das Signal-Rausch-Verhältnis. Besonders bei Minderperfusionen führt ein kleines Signal-Rausch-Verhältnis zu schlechten Ergebnissen oder es sind unter Umständen die Messungen auch gar nicht möglich. Bei sehr kleinen Perfusionen sind die Nutzsignale sehr schwach ausgeprägt. Da sie durch Wechselwirkung zwischen pulsierendem Blut und Licht entstehen, kann man die Perfusion beispielsweise durch Strahlungswärme vergrößern (siehe Laser-Dioden aus Figur 3), wie sie bei der frequenzstimulierten differenziellen MIR-Spektroskopie erfindungsgemäß zur Anwendung kommen soll. Eine Erhöhung der Anzahl der Photonen, die eine Wechselwirkung mit dem Gewebe haben können, wird erfindungsgemäß zur Signalverbesserung beitragen.

Das Modul mit den PPG-Sensoren (optischen Photoplethysmographie-Sensor), z.B. in Pos. 3 eigestellt, erlaubt so eine kontinuierliche Blutdruckmessung mit den Einsatz von Absoptions-, Transmissions-, und Reflexionssensoren. Soweit es sich um Absoptions- und/oder Transmissionsdetektionen handelt, befinden sich die Lichtquelle und der optische Sensor auf gegenüberliegenden Seiten des zu prüfenden Gewebes Pos.8/9. Ein Reflexionssensor weist auf gleicher Seite Lichtquelle und Empfänger auf und ein Transflexionssensor ist beiderseits mit einem Empfänger ausgestattet, wie aus Figur 3 ersichtlich. Je nachdem, weiche Vitaldaten zu ermitteln sind, kommen jeweils unterschiedliche Module des Bus-Sensor-Systems zur Anwendung.

In vorteilhafter Ausgestaltung handelt es sich bei den verwendeten Lichtquellen um Weisslicht LED's und/oder Laser Dioden, die sich wegen ihrer großen Lichtleistung, ihrer geringen Leistungsaufnahme und ihrer geringen Baugröße besonders eignen. Zudem ist hier eine allenfalls sehr geringe Erwärmung der Komponenten sichergestellt, was ansonsten eine Verfälschung der Messergebnisse und einen höheren Energiebedarf bedeuten würde.

Der oben aufgeführten Theorie entsprechend werden in den Modulen mit Vorteil Photodioden als Empfänger eingesetzt Pos. 2, wobei ebenso Alternativen in Form von Phototransistoren oder CCD-Detektoren Verwendung finden können.

Außerdem gestattet die heute möglich Höchstintegration der elektronischen Komponenten eine sehr geringe Bauform und Bauhöhe, sodass damit erst das erfindungsgemäß angestrebte Bus-Sensor-System mit wahlweise je nach Anwendungsfalls ausgestatteten Modulen das Prinzip eines flexiblen Bus-Sensor-Systems in Form eines tragbaren, mobilen, flexiblen und/oder halbstarren Bandes aus unterschiedlichstem Material Pos. 4 zulässt.

Wird das Bus-Sensor-System am Meßort des Körpers einer Person angebracht, so wird der zum Festhalten des Sensors verwendete Druck auf die Meßstelle über Drucksensoren Pos. 5 und über die Taxel-Folie Pos. 6 so eingestellt, dass so gut wie keine relativen Bewegungen zwischen Sensor und Probe stattfinden, gleichzeitig aber so klein, dass die durch die Pulswelle verursachte Volumenänderung mit dem Sensor nicht verhindert wird.

Ein weiterer Vorteil des Bus-Sensor-Systems sind zusätzlich wahlweise einsetzbare Module nach Pos. 2, die eine Positionsbestimmung über GPS und/oder Mobilefunk erlauben und/oder die Funktion eines kleinen mobilen Smartphones in der CPU Pos. 7 implizieren.

Die von dem Bus-Sensor-System ermittelten Daten auf dem Bus Pos. 1 werden in einem 4/8-Bit Standardformat über eine standardisierte Schnittstelle einem Dateisystem bereitgestellt, so dass über den mobilen Transfer der Daten eine Einspeisung der Messwerte in einen Datenbank-Server zur KIbasierten Auswertung und Diagnose möglich ist.

Alternativ können dem Bus-Sensor-System weitere Module mit Sensoren zugeordnet, die überwiegend im Alltag von Kliniken und Arztpraxen eingesetzt werden. Etwa Sensoren zur Ermittlung der venösen Sauerstoffsättigung, zur Erstellung von EKG oder EEG, ein Hautimpedanzsensor, Atemgassensoren und/oder chemische One-Chip-Lab-Sensoren für Tränen-, Gas-, Sekret- und Schweißanalysen.

## Patentansprüche

1. Multi-Bus-Sensor-System und Mobil-Modulares Verfahren zur nichtinvasiven Erfassung, Übertragung, Analyse und Diagnose des neurologischen und physiologischen Gesundheits-Zustandes einer erwachsenen Person auf der Grundlage von frequenzstimulierter optischer MIR Molekülspektroskopie, fraktionellen Sauerstoff-Sättigung, Hämoglobinkonzentrationen, Herzfrequenz, Blutdruck, Blutdruckvarianten, Temperatur, Herzfrequenzvariabilität und der Einkanal EKG's und EEG's zur Detektion von Standardabweichungen im Vergleich mit Standard- und/oder Vorlaufwerten, die sich auf einer Server-Datenbank befinden.

2. Bus-Sensor-System und Verfahren gemäß Anspruch 1, **dadurch gekennzeichnet, dass** die frequenzstimulierte optisch-tomografische MIR Molekülspektroskopie einen besonderen Schwerpunkt in der abgleichenden Konzentrationsmessung zur Erfassung von Hömoglobin und deren Stoffverbindungen, Myoglobin, Troponin I + T und Glukoseverbindungen hat.

3. Bus-Sensor-System und Verfahren gemäß einem der Ansprüche 1 oder 2, **dadurch gekennzeichnet, dass** eine frequenzmodulierte dermale Hautleitfähigkeit bestimmt werden kann und eine simultane nicht invasive Erfassung mehrerer Vital-Parameter in einem engen Zeitrahmen durchgeführt wird.

4. Bus-Sensor-System und Verfahren gemäß einem der vorhergehenden Ansprüche, **dadurch gekennzeichnet, dass** eine permanente Messung, Erfassung, Übertragung, Auswertung und Analyse auf einem dezentralen Server-System vorgenommen wird.

5. Bus-Sensor-System und Verfahren gemäß einem der vorhergehenden Ansprüche, **dadurch gekennzeichnet, dass** eine KI-basierte Daten-Analyse-Software aus den Vitaldaten eine objektivierte Diagnose des Gesundheitszustandes durchführen kann.

6. Bus-Sensor-System und Verfahren gemäß einem der Ansprüche 4 oder 5, **dadurch gekennzeichnet, dass** die gemessenen Parameter und die daraus ermittelten Ergebnisse, komprimiert etwa auf einem Display-Modul des Bus-Sensor-Systems dargestellt werden können.

7. Bus-Sensor-System und Verfahren gemäß vorhergehende Ansprüche **dadurch gekennzeichnet, dass** das Mess- und Verfahrensprinzip geeignet ist, als ein Frühwarnsystem für sich abzeichnende kardiovaskuläre und neurophysiologische Problem genutzt werden kann.

8. Bus-Sensor-System und Verfahren gemäß einem der Ansprüche 1 und 2, **dadurch gekennzeichnet, dass** die Bus-Sensor-Band-Vorrichtung aus mehreren über den Bus verbundene Modulen für verschiedene Anwendungen und Leistungen ausgestattet werden kann, wobei auch die Einbindung von weiteren am Körper fixierten Modulen über RFID-kommunikation möglich ist.

9. Bus-Sensor-System gemäß Anspruch 8, **dadurch gekennzeichnet, dass** ein Modul die Ausgestaltung eines Armbanduhrgehäuses haben kann und die Funktion einer Armbanduhr hat.

10. Bus-Sensor-System gemäß einem der Ansprüche 8 oder 9, **dadurch gekennzeichnet, dass** ein Modul als GPS-Empfänger zur simultanen Positionsbestimmung des Trägers arbeitet.

11. Bus-Sensor-System und Verfahren gemäß einem der Ansprüche 8 bis 10, **dadurch gekennzeichnet, dass** ein Modul als Smartphone fungiert und Telefonate und Positionsbestimmung über den Mobilfunk ermöglicht.

12. Bus-Sensor-System und Verfahren gemäß einem der Ansprüche 8 bis 11, **dadurch gekennzeichnet, dass** ein Modul als Geräusch- und Sound-Sensor ausgestaltet wird, um Umweltbelastungen im Höhr-Akustischen Spektrum zu ermitteln.

13. Bus-Sensor-System und Verfahren gemäß einem der Ansprüche 8 bis 12, **dadurch gekennzeichnet, dass** ein Modul als Gas-Sensor ausgestaltet ist, um Luftbelastungen zu messen.

14. Bus-Sensor-System und Verfahren gemäß einem der Ansprüche 8 bis 13, **dadurch gekennzeichnet, dass** alle Module für die Datenkommunikation auf den 8-Bit-Bus des System angekoppelt und/oder über eine RFID-kommunikation aufgeschaltet sind.

15. Bus-Sensor-System und Verfahren gemäß Anspruch 14, **dadurch gekennzeichnet, dass** die Datenkommunikation auf dem 8-Bit-Bus nach dem internationalen XT-ISO-Standard lesbar ist.

16. Bus-Sensor-System und Verfahren gemäß einem der Ansprüche 8 bis 15, **dadurch gekennzeichnet, dass** dem Datenbus ein ARM- und/oder ein anderer energieoptimierte Prozessor für das Datenhandling zugeordnet ist.

17. Bus-Sensor-System und Verfahren gemäß einem der Ansprüche 8 bis 16, **dadurch gekennzeichnet, dass** die Auswertung und der Transfer der Sensordaten durch die Prozessoreinheit online über das Internet auf einem gesicherten Kanal verschlüsselt durchführbar ist.

18. Bus-Sensor-System und Verfahren gemäß einem der Ansprüche 8 bis 17, **dadurch gekennzeichnet, dass** der Anpressdruck des Sensors-Systems auf den Messort dynamisch so einstellbar ist, dass er kleiner als der diastolische Blutdruck des Patienten ist und über die Gelgefüllten Blasen und/oder eine Taxel-Folie erfasst wird.

19. Bus-Sensor-System und Verfahren gemäß einem der Ansprüche 8 bis 17, **dadurch gekennzeichnet, dass** mindestens ein Modul als wechselbarer Akkumulator des System mit Energie versorgt.

20. Bus-Sensor-System und Verfahren gemäß dem der Anspruch 19, **dadurch gekennzeichnet, dass** die Ausführung des Moduls als Energie-Ernter und/oder als Solarmodul ausgestaltet sein kann.

21. Bus-Sensor-System und Verfahren gemäß einem der Ansprüche 1 bis 17, **dadurch gekennzeichnet, dass** als Bus-Träger verschiedene flexible oder nichtflexible Materialien zum Einsatz kommen können.

POS.6 TAXEL-FOLIE  POS.3 PPG  POS.7 CPU/DISPLAY  POS.1 BUS  POS.5 GEL-BLASEN

TEXEL-SYSTEM POS.4

MODULE FÜR SENSOREN/DIODEN/PRISMENFOLIE/CPU POS 2

Figur 1

QUERSCHNITT ARM, HANDGELENK BEIN

Figur 2

DIFFERENZIELLE HIR-SPEKROSKOPIE

PHOTODIODEN 2    2 LASER 1 LEUCHT DIODEN    PHOTODIODEN 1

PRISMEN    $I_a$    $I_a$    PRISMEN-FOLIE

$I_c$    $I_b$    $I_b$    $I_c$    k

PRISMEN WINKEL k

Figur 3

ABSORPTIONSSPEKTREN ABB.1

Abb.1

REFLEXIONSSPEKTREN ABB 2

Abb.2

Abb.3

Abb4

Europäisches Patentamt
European Patent Office
Office européen des brevets

## EUROPÄISCHER RECHERCHENBERICHT

Nummer der Anmeldung

EP 13 17 1362

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2012/150047 A1 (TERUMOTO KOJI [JP] ET AL) 14. Juni 2012 (2012-06-14) * Absätze [0103], [0106], [0109], [0117], [0120], [0121], [0123], [0126], [0128], [0130] * ----- | 1-19 | INV. A61B5/00 A61B5/0205 A61B5/1455 ADD. A61B5/024 |
| X | WO 2011/109716 A2 (NEUMITRA LLC [US]; GOLDBERG ROBERT [US]; YADAV SHAILENDRA [US]) 9. September 2011 (2011-09-09) * Abbildung 1 * * Seite 7, Zeilen 9-15 * * Seite 13, Zeilen 16-18 * * Seite 14, Zeilen 4-14 * * Seite 14, Zeilen 24-26 * * Seite 15, Zeilen 12-15 * * Seite 16, Zeile 8 - Seite 18, Zeile 14 * * Seite 18, Zeilen 15-23 * * Seite 19, Zeilen 27-28 * * Seite 20, Zeilen 3-5 * * Seite 20, Zeilen 16-18 * * Seite 24, Zeilen 1-3 * * Seite 28, Zeilen 27-28 * * Seite 29, Zeilen 3-14 * ----- | 1-19 | |
| X | WO 2011/034881 A1 (SOTERA WIRELESS INC [US]; MOON JIM [US]; VISSER HENK [US]; HUNT ROBERT) 24. März 2011 (2011-03-24) * Absätze [0007] - [0011], [0021] * ----- | 1-19 | RECHERCHIERTE SACHGEBIETE (IPC) A61B |
| X | US 2012/184827 A1 (SHWARTZ SHOULAMIT COHEN [IL] ET AL) 19. Juli 2012 (2012-07-19) * Abbildung 5A * * Absätze [0037] - [0040], [0046], [0073], [0104] - [0114], [0178] - [0181] * ----- -/-- | 1-19 | |

Der vorliegende Recherchenbericht wurde für alle Patentansprüche erstellt

| Recherchenort | Abschlußdatum der Recherche | Prüfer |
|---|---|---|
| Den Haag | 14. Mai 2014 | Albrecht, Ronald |

**Europäisches Patentamt**
**European Patent Office**
**Office européen des brevets**

# EUROPÄISCHER RECHERCHENBERICHT

Nummer der Anmeldung

EP 13 17 1362

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | WO 2012/170305 A1 (ALIPHCOM [US]; RAHMAN HOSAIN SADEQUR [US]; DRYSDALE RICHARD LEE [US];) 13. Dezember 2012 (2012-12-13)<br>* Seite 5, Zeilen 17-26 *<br>* Seite 6, Zeilen 10-15 *<br>* Seite 11, Zeile 4 *<br>* Seite 15, Zeilen 13-15 *<br>* Seite 16, Zeile 37 - Seite 17, Zeile 3 *<br>* Seite 17, Zeilen 11-12 *<br>* Seite 17, Zeile 26 *<br>* Seite 27, Zeilen 21-25 *<br>----- | 1-19 | |
| A | US 2006/254369 A1 (YOON EUISIK [US] ET AL) 16. November 2006 (2006-11-16)<br>* Abbildung 1B *<br>* Absätze [0002], [0026], [0062], [0081] *<br>----- | 18 | |
| | | | **RECHERCHIERTE SACHGEBIETE (IPC)** |

Der vorliegende Recherchenbericht wurde für alle Patentansprüche erstellt

| Recherchenort | Abschlußdatum der Recherche | Prüfer |
|---|---|---|
| Den Haag | 14. Mai 2014 | Albrecht, Ronald |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
................................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT**
**ÜBER DIE EUROPÄISCHE PATENTANMELDUNG NR.**

EP 13 17 1362

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-05-2014

| Im Recherchenbericht angeführtes Patentdokument | Datum der Veröffentlichung | Mitglied(er) der Patentfamilie | Datum der Veröffentlichung |
|---|---|---|---|
| US 2012150047 A1 | 14-06-2012 | CN 102551686 A<br>US 2012150047 A1 | 11-07-2012<br>14-06-2012 |
| WO 2011109716 A2 | 09-09-2011 | EP 2542147 A2<br>KR 20130051922 A<br>US 2011245633 A1<br>WO 2011109716 A2 | 09-01-2013<br>21-05-2013<br>06-10-2011<br>09-09-2011 |
| WO 2011034881 A1 | 24-03-2011 | EP 2470067 A1<br>SG 179149 A1<br>WO 2011034881 A1 | 04-07-2012<br>27-04-2012<br>24-03-2011 |
| US 2012184827 A1 | 19-07-2012 | US 2012184827 A1<br>WO 2010146588 A2 | 19-07-2012<br>23-12-2010 |
| WO 2012170305 A1 | 13-12-2012 | CA 2822708 A1<br>EP 2718918 A1<br>WO 2012170305 A1 | 13-12-2012<br>16-04-2014<br>13-12-2012 |
| US 2006254369 A1 | 16-11-2006 | KEINE | |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6226540 B

**In der Beschreibung aufgeführte Nicht-Patentliteratur**

- Global atlas. *Cardiovascular diseases,* 29. September 2012, ISBN 9789241564373
- **STRAUS SE ; MAJUMDAR SR ; MC ALISTER FA.** New evidence for stroke prevention. Scientific Review. *JAMA,* 2002, vol. 288, 1388-95
- *Quelle: Deutsche Hochdruckliga e.V. Leitlinien zur Behandlung der Arteriellen Hypertonie,* 2008, 9, www.hochdruck-liga.de
- **QUELLE: MANCIA G et al.** Guidelines for the management of arterial hypertension. The Task Force for the Management of Arterial Hypertension of the European Society of Hypertension (ESH) and of the European Society of Cardiology (ESC. *European Heart Journal,* 2007, vol. 28, 1462-1536
- **QUELLE: HEROLD, G.** Innere Medizin. *Eine vorlesungsorientierte Darstellung Herold,* 2009
- **P.LASCH ; J. KNEIPP.** Biomedical Vibrational Spectroscopy. JohnWiley&Sons, 2008
- **ALEXANDER CM ; TELLER LE ; GROSS JB.** Principles of pulse oximetry: theoretical and practical considerations. *Anesth Analg,* 1989, vol. 68 (3), 368-76
- **SCHMITT JM.** Simple photon diffusion analysis of the effects of multiple scattering on pulse oximetry. *IEEE Trans Biomed Eng,* 1991, vol. 38 (12), 1194-203
- **SUZUKI S ; TAKASAKI S ; OZAKI T ; KOBAYASHI Y.** Tissue oxygenation monitor using NIR spatially resolved spectroscopy. *Proc. SPIE,* April 1999
- **W. WU.** Science Express, 15.4.3013. *Physik Journal,* Juni 2013, 14